# EUROPEAN PATENT SPECIFICATION

(11) **EP 3 773 433 B1**
(45) Date of publication and mention of the grant of the patent: **13.10.2021**
(21) Application number: 19708323.1
(22) Date of filing: 06.03.2019
(51) Int. Cl.: A61K 8/33, A61K 8/34, A61K 8/37, A61K 8/58, A61Q 15/00, A61K 8/891, A61K 8/02

(54) **NON-ALUMINIUM ANTIPERSPIRANT COMPOSITIONS**
ALUMINIUMFREIE SCHWEISSHEMMENDE ZUSAMMENSETZUNGEN
COMPOSITIONS ANTISUDORIFIQUES SANS ALUMINIUM

(30) Priority: 28.03.2018 EP 18164560
(43) Date of publication of application: 17.02.2021
(73) Proprietor: Unilever IP Holdings B.V., 3013 AL Rotterdam (NL); Unilever Global IP Limited, Wirral, CH62 AZD (GB)
(72) Inventor: ZDRAVKOVA, Aneliya, Nikolova, Wirral Merseyside CH63 3JW (GB)
(74) Representative: Whaley, Christopher
(86) International application number: PCT/EP2019/055514
(87) International publication number: WO 2019/185314

(56) References cited:
- WO-A1-94/24993
- GB-A- 2 273 872
- US-A1- 2008 267 895

## Description

### Field of Invention

The present invention is in the field of cosmetic compositions and their use as antiperspirants, in particular, non-aluminium antiperspirant compositions.

### Background

EP 550,960 A1 (Unilever, 1992) discloses the use as an antiperspirant active of an amphiphilic material which forms, upon contact with perspiration, a water-insoluble liquid crystal phase of greater than one dimensional periodicity. This publication does not disclose ethanolic compositions, nor their stability issues, nor their use in aerosol compositions.

WO 94/024993 (Unilever, 1994) discloses an antiperspirant composition comprising an amphiphilic material which forms, upon contact with perspiration, a water-insoluble liquid crystal phase of greater than one dimensional periodicity, in a cosmetic vehicle comprising a volatile silicone and containing less than 10% by weight of the total composition of a short chain monohydric alcohol. This publication does not disclose compositions with the specific ratios of components as defined herein and does not disclose their importance in the low temperature (0°C) stability thereof.

### Summary of Invention

It is an object of the present invention to provide an antiperspirant composition that does not require the presence of an aluminium or zirconium salt to deliver an antiperspirancy benefit. It is a further of the present invention to do this from a composition that has a high degree of storage stability, particularly at low temperatures.

It is an object of the present invention to provide a base for an antiperspirant aerosol composition, the base being free from aluminium salts and having good stability, particularly at low temperature. It is a further of the present invention to provide a stable antiperspirant aerosol composition that does not require the presence of an aluminium or zirconium salt to deliver an antiperspirancy benefit.

In a first aspect of the invention, there is provided an antiperspirant composition comprising ethanol, amphiphilic material and volatile silicone, the amphiphilic material being a mixture consisting of isostearyl alcohol and glycerol monolaurate at a ratio of from 25:75 to 45:55 by weight, wherein:
(i) the ratio of amphiphilic material to (ethanol + volatile silicone) is greater than or equal to 1:9 by weight;
(ii) the ratio of volatile silicone to (ethanol + amphiphilic material) is from 1:9 to 1:1 by weight and wherein
the ratio of ethanol to amphiphilic material is greater than or equal to 7:3 by weight.

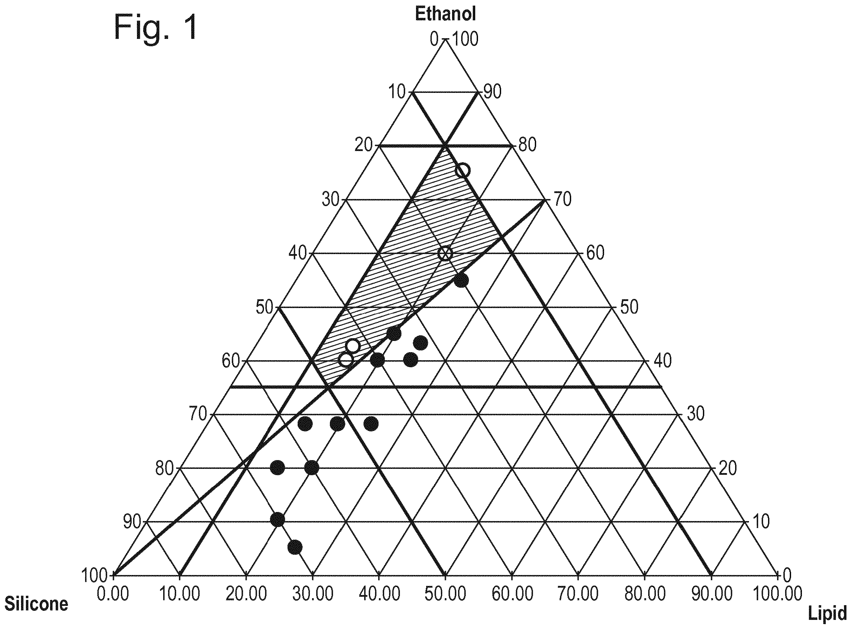

The three-component "formulation space" indicated in the first aspect of the invention is represented by the shaded are on the ternary phase diagram shown as Figure 1.

In a second aspect of the invention, there is provided a method of manufacture of an antiperspirant aerosol composition comprising the addition of volatile propellant to a composition according to the first aspect of the invention.

In a third aspect of the invention, there is provided a cosmetic method of attaining an antiperspirant benefit comprising the topical application of a composition according to the first aspect of the invention.

### Detailed Description

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, "ambient conditions" refers to 25°C and 1 atmosphere pressure, unless otherwise indicated.

Herein, all percentages, ratios and amounts are by weight, unless otherwise indicated.

Herein, the word "comprising" is intended to mean "including" but not necessarily "consisting of", i.e., it is non-exhaustive.

Herein, "cosmetic" methods and compositions should be understood to mean non-therapeutic methods and compositions, respectively.

Herein, an "amphiphilic material" is a material defined by having both hydrophilic and hydrophobic portions in its structure.

Herein, "water-insoluble" means having a solubility in water of less than 0.1% by weight (at 37°C).

The compositions of the invention are particularly effectively when applied to the underarm regions of the human body and/or the feet. The compositions are especially effectively when applied to the underarm regions of the human body.

Antiperspirant aerosol compositions consist of a volatile propellant and a base. The components of the base are typically mixed together first and the volatile propellant is added last in a process sometimes called "gassing". It is important that the base has good storage stability because there can be a significant period between the preparation of the base and the addition of the propellant.

Herein, the "base" of an antiperspirant aerosol composition is all the components of the total composition other than the volatile propellant.

It is important that the fully formulated antiperspirant aerosol composition has good storage stability, so that it can survive prolonged transit to stores and extended periods on shelf prior to purchase and use.

The present invention involves base formulations having superior storage stability, particularly at low temperatures, such as 0°C. By achieving base stability at low temperatures, the present invention enables the delivery of fully formulated antiperspirant aerosol compositions having good storage stability over prolonged periods.

The amphiphilic material is a mixture of isostearyl alcohol and glycerol monolaurate, at a weight ratio of from 25: 75 to 45: 55.

It is essential to have sufficient of the amphiphilic material present in the composition to achieve an acceptable degree of antiperspirancy when the composition is applied to the skin of the human body. The amphiphilic material is at least 10% of the tri-component mixture consisting of ethanol, amphiphilic material and volatile siloxane.

The maximum content of amphiphilic material in the tri-component mixture consisting of ethanol, amphiphilic material and volatile siloxane is from 15% to 27%, these figures being calculable from the other essential requirements of the invention.

The amphiphilic material serves as the antiperspirant active for the composition.

According to a preferred embodiment of the invention, the amphiphilic material physically swells as it forms the liquid crystal structure on contact with perspiration, hence enhancing the pore-blocking effect.

Antiperspirant compositions according to the present invention are preferably free from aluminium or zirconium antiperspirant salts.

The content of amphiphilic material in the total composition, ignoring any volatile propellant therein, is preferably at least 10%, more preferably at least 12% and most preferably at least 15%.

Herein, a "volatile silicone" is a silicone having a vapour pressure of greater than 1 Pa at 25°C.

The volatile silicone and its level of incorporation serves to reduce potential irritation and/or promote the good sensory properties of the composition.

The volatile silicone is from 10% to 50% of the tri-component mixture consisting of ethanol, amphiphilic material and volatile siloxane.

The content of volatile silicone in the total composition, ignoring any volatile propellant therein, is preferably from 10% to 50%, more preferably from 15% to 50% most preferably from 15% to 45%.

It is preferred that the volatile silicone comprises greater than 90% by weight of, or consists of siloxanes having from 2 to 6 silicone atoms, arranged in either a cyclic or linear fashion.

Linear siloxanes have the general formula: Me₃SiO(Me₂SiO)ₙSiMe₃, where Me = methyl group (-CH₃).
When n = 0, the siloxane is hexamethyldisiloxane.
When n = 1, the siloxane is octamethyltrisiiloxane.
When n = 2, the siloxane is decamethyltetrasiloxane.
When n = 3, the siloxane is dodecamethylpentasiloxane.

It is particularly preferred that the volatile silicone comprises greater than 90% by weight of, or consists of, siloxanes selected from the group consisting of hexamethyldisiloxane, octamethyltrisiiloxane, decamethyltetrasiloxane, dodecamethylpentasiloxane and decamethylcyclopentasiloxane.

It is especially preferred that the volatile silicone comprises greater than 90% by weight of, or consists of, siloxanes selected from the group consisting of hexamethyldisiloxane, octamethyltrisiiloxane, decamethyltetrasiloxane and decamethylcyclopentasiloxane.

The purpose of the ethanol is principally to solubilise the amphiphilic material. The ethanol is present at from 35% to 80% of the tri-component mixture consisting of ethanol, amphiphilic material and volatile siloxane, these figures being calculable from the other essential requirements of the invention.

The content of ethanol in the total composition, ignoring any volatile propellant therein, is preferably at least 10%, more preferably at least 12% and most preferably at least 15%.

The ratio of ethanol to amphiphilic material must be at least 7: 3 or, more precisely, 70: 30. More preferably this ratio is at least 71: 27 and most preferably at least 72: 28.

In preferred embodiments of the invention, the composition is an antiperspirant aerosol composition comprising a volatile propellant. In such compositions, the volatile propellant preferably comprises from 35 to 95% of the total composition, more preferably from 50 to 90% and most preferably from 65 to 90% of the total composition.

When employed, the propellant is commonly either a compressed gas or a material that boils at below ambient temperature, preferably at below 0°C, and especially at below -10°C. Examples of compressed gasses include nitrogen and carbon dioxide. Examples of low boiling point materials include dimethyl ether. Other possible low boiling point materials that may be used as volatile propellants are hydrofluorocarbons containing from 2 to 4 carbons, at least one hydrogen and 3 to 7 fluorine atoms.

In certain preferred embodiments involving antiperspirant aerosol compositions, the volatile propellant used comprises or is solely dimethyl ether.

When compositions according to the invention are aerosol compositions, they can be made in a conventional manner by first preparing a base composition, charging the base composition into the aerosol can, fitting a valve assembly into the mouth of the can, thereby sealing the can, and thereafter charging volatile propellant into the can to a desired pressure, and finally fitting an actuator on or over the valve assembly.

A preferred additional component of compositions of the invention is a deodorant active. These are typically antimicrobial agents active against bacterial on the skin of the human body. These serve to reduce malodour and especially useful in compositions in which the amphiphilic material is not itself an antimicrobial agent.

When employed, the level of incorporation is preferably 0.01%-5%, more preferably from 0.01-2% and most preferably from 0.03%-0.5% by weight of the total composition.

Preferred anti-microbial deodorant agents are those that are more efficacious than simple alcohols such as ethanol. Particularly preferred anti-microbial deodorant agents are soluble in ethanol, meaning that they a solubility in ethanol of at least 10g/L at 20°C.

Examples of suitable anti-microbial deodorant agents include niacinamide; quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals, 2',4,4'-trichloro,2-hydroxy-diphenyl ether (triclosan), 3,7,11-trimethyldodeca-2,6,10-trienol (farnesol), essential oils such as Tea Tree Oil and Thyme Oil, climbazole, octapyrox, ketoconazole, zinc pyrithione and mixtures thereof.

A preferred optional component is a preservative, such as ethyl or methyl parabens or BHT (butyl hydroxy toluene), typically in an amount of from 0.01 to 0.1% by weight of the total composition.

The invention will now be described by some examples, which do not limit the extent of the invention.

### Examples

Numerous examples and comparative examples have been prepared and tested. Without exception, the examples according to the invention performed better than the comparative examples.

The volatile silicones used in these examples were:
DC245 = cyclopentasiloxane and
DC200 (1.5 cS) = decamethyltetrasiloxane. Designated as DC200 in Table 1.

These volatile silicones were often used as a 15: 85 blend of DC245 and DC200 (1.5 cS), designated as B1 in Table 1. Each of these materials is available from Dow Corning.

The amphiphilic material used in these examples is designated "lipid" and was a 60:40 blend of glycerol monolaurate and isostearyl alcohol.

The ethanol used in these examples was absolute alcohol.

The compositions indicated in Table 1 were prepared by methods known in the art. The storage stability at 0°C and ambient temperature* was assessed after 6 weeks.

* In this experiment, the ambient temperature ranged between 14°C and 22°C, resulting in some slight inconsistency in the stability results obtained.

Instability manifested itself by phase separation, the lipid component(s) typically crystallising out of the liquid phase.

Figure 1 is a ternary phase diagram representing the "tri-component" compositions detailed in Table 1. In this Figure, the examples according to the invention are indicated by hollow dots and comparative examples are indicated by filled dots.

The examples according to the invention were found to exhibit superior storage stability at 0°C.

**Table 1**

| Example | Ethanol | Volatile silicone | | Lipid | Stability at 0°C | Stability at ambient |
|---|---|---|---|---|---|---|
| | % w/w | % w/w | Nature | % w/w | | |
| A | 28 | 57 | B1 | 15 | No | Yes |
| B | 28 | 52 | B1 | 20 | No | Yes |
| C | 20 | 65 | B1 | 15 | No | No |
| C2 | 20 | 65 | DC200 | 15 | No | No |
| 1 | 40 | 45 | B1 | 15 | **YES** | Yes |
| 2 | 44 | 41 | B1 | 15 | **YES** | Yes |
| 3 | 75 | 10 | B1 | 15 | **YES** | Yes |
| D | 44 | 41 | DC200 | 15 | No | No |
| E | 28 | 47 | B1 | 25 | No | No |
| E2 | 28 | 47 | B1 | 25 | No | No |
| E3 | 43 | 32 | B1 | 25 | No | No |
| F | 20 | 60 | B1 | 20 | No | No |
| G | 40 | 40 | B1 | 20 | No | Yes |
| G2 | 45 | 35 | B1 | 20 | No | Yes |
| G3 | 45 | 35 | B1 | 20 | No | Yes |
| 4 | 60 | 20 | B1 | 20 | **YES** | Yes |
| H | 10 | 70 | B1 | 20 | No | No |
| J | 5 | 70 | B1 | 25 | No | No |
| K | 40 | 35 | B1 | 25 | No | Yes |
| K2 | 40 | 35 | DC200 | 25 | No | No |
| K3 | 55 | 20 | B1 | 25 | No | No |
| K4 | 55 | 20 | DC200 | 25 | No | Yes |

### Examples 5 to 13

The aerosol compositions indicated in Table 2 were prepared by using the indicated composition from Table 1 as a "base" composition and adding DME as propellant by methods known in the art. Three different base to propellant ratios were tested: 65: 35, 50: 50 and 35: 65. The samples were stored in pressurised glass aerosol containers and their stability was assessed after 24 hours, but otherwise in same manner as for the base compositions in the previous test.

All the compositions proved to be stable at both ambient temperature and 0°C for the duration of the test (24 hours).

**Table 2**

| Component | **Example** | | | | | | | | |
|---|---|---|---|---|---|---|---|---|---|
| | **5** | **6** | **7** | **8** | **9** | **10** | **11** | **12** | **13** |
| Base 1 | 65 | 50 | -- | -- | -- | -- | -- | -- | -- |
| Base 2 | -- | -- | 65 | 50 | 35 | -- | -- | -- | -- |
| Base 3 | -- | -- | -- | -- | -- | 65 | 50 | 35 | -- |
| Base 4 | -- | -- | -- | -- | -- | -- | **--** | -- | 35 |
| DME | 35 | 50 | 35 | 50 | 65 | 35 | 50 | 65 | 65 |

## Claims

1. An antiperspirant composition comprising ethanol, amphiphilic material and volatile silicone having a vapour pressure of greater than 1 Pa at 25°C, the amphiphilic material being a mixture consisting of isostearyl alcohol and glycerol monolaurate at a ratio of from 25: 75 to 45: 55 by weight, **characterised in that**:
(i) the ratio of amphiphilic material to (ethanol + volatile silicone) is greater than or equal to 1:9 by weight;
(ii) the ratio of volatile silicone to (ethanol + amphiphilic material) is from 1:9 to 1:1 by weight and wherein
(iii) the ratio of ethanol to amphiphilic material is greater than or equal to 7:3 by weight.

2. A composition according to claim 1, wherein the composition is an aerosol composition comprising a volatile propellant.

3. A composition according to claim 2, wherein the volatile propellant is dimethyl ether (DME).

4. A composition according to claim 1 or claim 2, wherein the volatile propellant is present at from 30 to 90% by weight of the composition.

5. A composition according to any of the preceding claims, wherein the content of the amphiphilic material in the total composition, ignoring any volatile propellant therein, is at least 10% by weight.

6. A composition according to claim 5, wherein the content of the amphiphilic material in the total composition, ignoring any volatile propellant therein, is at least 15% by weight.

7. A composition according to any of the preceding claims, wherein the content of volatile silicone in the total composition, ignoring any volatile propellant therein, is from 10% to 50% by weight.

8. A composition according to any of the preceding claims, wherein the content of ethanol in the total composition, ignoring any volatile propellant therein, is at least 10% by weight.

9. A composition according to any of the preceding claims, wherein the ratio of ethanol to amphiphilic material is at least 72: 28.

10. A composition according to any of preceding claims, wherein the volatile silicone comprises greater than 90% by weight of siloxanes selected from the group consisting of hexamethyldisiloxane, octamethyltrisiiloxane, decamethyltetrasiloxane, dodecamethylpentasiloxane and decamethylcyclopentasiloxane.

11. A composition according to claim 7, wherein the volatile silicone comprises greater than 90% by weight of siloxanes selected from the group consisting of hexamethyldisiloxane, octamethyltrisiiloxane, decamethyltetrasiloxane and decamethylcyclopentasiloxane.

12. A composition according to any of preceding claims, which is free from aluminium or zirconium antiperspirant salts.

13. A composition according to any of preceding claims, comprising an antimicrobial agent.

14. A cosmetic method of attaining an antiperspirant benefit comprising the topical application of a composition according to any of claims 1 to 13.

## Patentansprüche

1. Antitranspirantzusammensetzung, umfassend Ethanol, amphiphiles Material und flüchtiges Silicon mit einem Dampfdruck von mehr als 1 Pa bei 25 °C, wobei das amphiphile Material eine Mischung bestehend aus Isostearylalkohol und Glycerinmonolaurat in einem Gewichtsverhältnis von 25:75 bis 45:55 ist, **dadurch gekennzeichnet, dass**:
(i) das Verhältnis von amphiphilem Material zu (Ethanol + flüchtigem Silicon), bezogen auf das Gewicht, größer als oder gleich 1:9 ist;
(ii) das Verhältnis von flüchtigem Silicon zu (Ethanol + amphiphilem Material), bezogen auf das Gewicht, von 1:9 bis 1:1 ist und wobei:
(iii) das Verhältnis von Ethanol zu amphiphilem Material, bezogen auf das Gewicht, größer als oder gleich 7:3 ist.

2. Zusammensetzung nach Anspruch 1, wobei die Zusammensetzung eine Aerosolzusammensetzung ist, die ein flüchtiges Treibmittel umfasst.

3. Zusammensetzung nach Anspruch 2, wobei das flüchtige Treibmittel Dimethylether (DME) ist.

4. Zusammensetzung nach Anspruch 1 oder Anspruch 2, wobei das flüchtige Treibmittel mit 30 bis 90 Gewichts-% der Zusammensetzung vorhanden ist.

5. Zusammensetzung nach irgendeinem der vorhergehenden Ansprüche, wobei der Gehalt des amphiphilen Materials in der Gesamtzusammensetzung, ohne Berücksichtigung jeglicher flüchtigen Treibmittel darin, mindestens 10 Gewichts-% beträgt.

6. Zusammensetzung nach Anspruch 5, wobei der Gehalt des amphiphilen Materials in der Gesamtzusammensetzung, ohne Berücksichtigung jeglicher flüchtigen Treibmittel darin, mindestens 15 Gewichts-% beträgt.

7. Zusammensetzung nach irgendeinem der vorhergehenden Ansprüche, wobei der Gehalt an flüchtigem Silicon in der Gesamtzusammensetzung, ohne Berücksichtigung jeglicher flüchtigen Treibmittel darin, 10 bis 50 Gewichts-% beträgt.

8. Zusammensetzung nach irgendeinem der vorhergehenden Ansprüche, wobei der Gehalt an Ethanol in der Gesamtzusammensetzung, ohne Berücksichtigung jeglicher flüchtigen Treibmittel darin, mindestens 10 Gewichts-% beträgt.

9. Zusammensetzung nach irgendeinem der vorhergehenden Ansprüche, wobei das Verhältnis von Ethanol zu amphiphilem Material mindestens 72:28 beträgt.

10. Zusammensetzung nach irgendeinem der vorhergehenden Ansprüche, wobei das flüchtige Silicon mehr als 90 Gewichts-% Siloxane, ausgewählt aus der Gruppe bestehend aus Hexamethyldisiloxan, Octamethyltrisiloxan, Decamethyltetrasiloxan, Dodecamethylpentasiloxan und Decamethylcyclopentasiloxan, umfasst.

11. Zusammensetzung nach Anspruch 7, wobei das flüchtige Silicon mehr als 90 Gewichts-% Siloxane, ausgewählt aus der Gruppe bestehend aus Hexamethyldisiloxan, Octamethyltrisiloxan, Decamethyltetrasiloxan und Decamethylcyclopentasiloxan, umfasst.

12. Zusammensetzung nach irgendeinem der vorhergehenden Ansprüche, die frei von schweißhemmenden Aluminium- oder Zirconiumsalzen ist.

13. Zusammensetzung nach irgendeinem der vorhergehenden Ansprüche, umfassend ein antimikrobielles Mittel.

14. Kosmetisches Verfahren zur Erzielung eines schweißhemmenden Nutzens, umfassend die topische Anwendung einer Zusammensetzung nach irgendeinem der Ansprüche 1 bis 13.

## Revendications

1. Composition d'antiperspirant comprenant de l'éthanol, un matériau amphiphile et du silicone volatil ayant une pression de vapeur supérieure à 1 Pa à 25°C, le matériau amphiphile étant un mélange consistant en alcool isostéarylique et monolaurate de glycérol à un rapport de 25:75 à 45:55 en masse, **caractérisée en ce que** :
(i) le rapport de matériau amphiphile à (éthanol + silicone volatil) est supérieur ou égal à 1:9 en masse ;
(ii) le rapport de silicone volatil à (éthanol + matériau amphiphile) est de 1:9 à 1:1 en masse et dans laquelle
(iii) le rapport d'éthanol à matériau amphiphile est supérieur ou égal à 7:3 en masse.

2. Composition selon la revendication 1, dans laquelle la composition est une composition d'aérosol comprenant un propulseur volatil.

3. Composition selon la revendication 2, dans laquelle le propulseur volatil est du diméthyléther (DME).

4. Composition selon la revendication 1 ou revendication 2, dans laquelle le propulseur volatil est présent à de 30 à 90 % en masse de la composition.

5. Composition selon l'une quelconque des revendications précédentes, dans laquelle la teneur du matériau amphiphile dans la composition totale, ignorant tout propulseur volatil dans celle-ci, est d'au moins 10 % en masse.

6. Composition selon la revendication 5, dans laquelle la teneur du matériau amphiphile dans la composition totale, en ignorant tout propulseur volatil dans celle-ci, est d'au moins 15 % en masse.

7. Composition selon l'une quelconque des revendications précédentes, dans laquelle la teneur de silicone volatil dans la composition totale, ignorant tout propulseur volatil dans celle-ci, est de 10 % à 50 % en masse.

8. Composition selon l'une quelconque des revendications précédentes, dans laquelle la teneur d'éthanol dans la composition totale, ignorant tout propulseur volatil dans celle-ci, est d'au moins 10 % en masse.

9. Composition selon l'une quelconque des revendications précédentes, dans laquelle le rapport d'éthanol à matériau amphiphile est d'au moins 72:28.

10. Composition selon l'une quelconque des revendications précédentes, dans laquelle le silicone volatil comprend plus de 90 % en masse de siloxanes choisis dans le groupe consistant en hexaméthyldisiloxane, octaméthyltrisiloxane, décaméthyltétrasiloxane, dodécaméthylpentasiloxane et décaméthylcyclopentasiloxane.

11. Composition selon la revendication 7, dans laquelle le silicone volatil comprend plus de 90 % en masse de siloxanes choisis dans le groupe consistant en hexaméthyldisiloxane, octaméthyltrisiloxane, décaméthyltétrasiloxane et décaméthylcyclopentasiloxane.

12. Composition selon l'une quelconque des revendications précédentes, qui est exempte d'aluminium ou de sels d'antiperspirants de zirconium.

13. Composition selon l'une quelconque des revendications précédentes, comprenant un agent antimicrobien.

14. Procédé cosmétique atteignant un bénéfice d'antiperspirant comprenant l'application topique d'une composition selon l'une quelconque des revendications 1 à 13.
